(19) **Europäisches Patentamt / European Patent Office / Office européen des brevets**

(11) **EP 2 097 063 B1**

(12) **EUROPEAN PATENT SPECIFICATION**

(45) Date of publication and mention
of the grant of the patent:
**07.08.2013 Bulletin 2013/32**

(51) Int Cl.:
*A61K 47/32* (2006.01)   *A61K 31/565* (2006.01)
*A61P 15/00* (2006.01)   *A61P 15/08* (2006.01)

(21) Application number: **07822711.3**

(86) International application number:
**PCT/EP2007/062514**

(22) Date of filing: **19.11.2007**

(87) International publication number:
**WO 2008/061963 (29.05.2008 Gazette 2008/22)**

(54) **HELICALLY-SHAPED DRUG DELIVERY SYSTEM**

SPIRALFÖRMIGES ARZNEIMITTELABGABESYSTEM

SYSTÈME D'ADMINISTRATION DE MÉDICAMENTS DE FORME HÉLICOIDALE

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK RS**

(30) Priority: **20.11.2006 EP 06124377**

(43) Date of publication of application:
**09.09.2009 Bulletin 2009/37**

(73) Proprietor: **Intervet International B.V.
5831 AN Boxmeer (NL)**

(72) Inventors:
• **DRIANCOURT, Marc-Antoine
49071 Beaucouze (FR)**
• **DE GRAAFF, Wouter
5340 BH OSS (NL)**

• **BUTTAFOCO, Laura
5340 BH Oss (NL)**
• **PAYOT, Fabrice
49071 Angers (FR)**
• **VEENSTRA, Harm
5340 BH Oss (NL)**
• **VOSS, René
5340 BH Oss (NL)**

(74) Representative: **Stumm, Karin et al
Merck Sharp & Dohme Ltd.
Hertford Road
Hoddesdon, Hertfordshire
EN11 9BU (GB)**

(56) References cited:
**WO-A-2004/103336    GB-A- 1 119 674
US-A- 3 892 238    US-A1- 2006 051 391**

Printed by Jouve, 75001 PARIS (FR)

**Description**

The present invention relates to a helically-shaped medicated veterinary system suitable for delivery of a drug to the vaginal cavity of a non-human mammal and to a method of manufacture.

Drug delivery systems for insertion in the vagina are known in the art. US 4,237,885 discloses a rate-controlled drug delivery system comprising a tubular member twined about itself to form a multiplicity of continuous, entwined, mated members, wherein the pair of ends are joined to make a closed curved device.

The retention rate of intra-vaginal sponges with altrenogest for mares is described by Palmer in Journal of Reproduction and Fertility, suppl. 27 (1979), 263-270.

WO 9740776 discloses an intra-vaginal, variable geometry device (CIDR®), for use in cattle, sheep, deer and goats, comprising a matrix of a cured silicone rubber comprising more than 5% by weight of progesterone to the weight of the matrix and an exterior surface of 75 cm$^2$ or more contactable with the vaginal membrane and/or vaginal fluids.

A coil for locally dispensing medication to mammalian tissue is described in WO2004/105854. The coil is formed from a length of flexible tubing sealed at opposite ends and containing a drug.

A number of vaginal rings are known in the art. For example, US 4,292,965 discloses an intravaginal ring for use as a contraceptive comprising an inert elastomer core, a medicated layer encircling the core, and an outer inert elastomer layer and a method of manufacturing said intravaginal ring. Intravaginal rings made of silicone rubber and comprising levonorgestrel and 17β-estradiol are exemplified therein.

Further, EP 0876815 discloses a vaginal ring which is designed for the simultaneous release of a progestogenic steroid compound and an estrogenic steroid compound in a fixed physiological ratio over a prolonged period of time. The drug delivery system comprises at least one compartment comprising a thermoplastic polymer core containing the mixture of the progestogenic and estrogenic compounds and a thermoplastic polymer skin, the progestogenic compound being initially dissolved in the polymer core material in a relatively low degree of supersaturation. The preferred thermoplastic polymers are ethylene-vinyl acetate (EVA) copolymers. This contraceptive vaginal ring is marketed under the trademark Nuvaring® by Organon, the Netherlands.

WO2004/103336 discloses a vaginal ring comprising a thermoplastic polymer core comprising a pharmaceutically active compound in dissolved form, a thermoplastic intermediate layer comprising crystals of a first pharmaceutically active compound and a second pharmaceutically active compound in dissolved form, and a non medicated thermoplastic skin covering the intermediate layer.

A vaginal insert for treating disease is disclosed in US2003/0149334, comprising a body formed from a flexible material permitting the body to be coiled into a coiled state, to form a cylindrical configuration, allowing the insert to expand thereby contacting and pressing against the interior walls of the vagina. It is used for controlled and sustained delivery of drug for the treatment of diseases inside or outside the genital tract.

A system for vaginal insertion in horses, using especially designed treatment pads that are attached to a 'wishbone' carrier system, is described to have two S-shaped arms that can flex while inserted in the vagina resulting in sufficient tension to ensure that the device is retained during the whole treatment period (Cue Mare®). The system consistently generated vaginal irritation (J.B., Grimmet, Theriogenology 58 (2002) 585-587).

Further, U.S. 3,892,238 describes a helically-shaped drug supporting anchor (PRID) for insertion and retention in body cavities including a drug support surface with a spiral configuration for supporting a drug to be administered, and the combination of a drug supporting anchor as described above with a drug supported thereon in either strip form or as a uniform layer. The drug supporting anchor retains in the vagina with a helical configuration having a diameter greater than the diameter during insertion. The document describes that the locking of the spaced coils into the tissue is required for keeping the anchor in a well-defined position. The anchor exercises a continuous pressure on the vaginal wall to resist expelling thereof and to provide retention in the body cavity to facilitate release of a drug carried thereby.

Vaginitis and large purulent discharges have been detected in a significant number of mares at device removal (Dusart, P. et al (2006). Proc 9th congress of the World Equine Veterinary Association, Marrakech, pp 239-241; Handler, J., et al. (2006). Theriogenology 65, 1145-1158). It should be noted that U.S. 3,892,238 does not disclose the use of a system for delivery of a drug comprising a medicated fibre.

It is an object of the present invention to provide a drug delivery system of which the release rate can be controlled to the requirements of a variety of female non-human mammals and various therapeutic and zoo-technical indications.

It is a further object of the invention to provide a system that can be easily and cautiously inserted in the vaginal cavity of the female non-human mammal and easily removed.

It is still a further object of the present invention to provide a veterinary drug delivery system that displays high retention rates during treatment periods of one or more

US 2006/0051391 discloses a device for controlled intravaginal administration that includes an anchoring medium and a substance support medium.

weeks and high tolerability, i.e. prevention of vaginal tissue irritation and inflammation.

It is even a further object of the invention to provide a drug delivery system for female non-human mammals that is easy to manufacture and that allows fine-tuning the release rate of the system to the weight of the mammal and to effective blood levels related to the therapeutic or zoo-technical indication by cutting, prior to insertion, the spring to a predetermined length.

It is still a further object of the present invention to provide a veterinary system with the ability to tune the release rate without affecting the mechanical properties of the spring by adjusting the length of the system.

Furthermore, it is an object of the present invention to provide a veterinary system with a high ability to include a range of veterinary drugs and a high efficiency in delivering its drug.

Even further, it is an objective of the present invention to provide a delivery system that can have low to high drug loading, and which can deliver the drugs at a controlled and useful rate over prolonged periods of time.

Furthermore, it is an object of the present invention to provide a delivery system which can deliver the drugs at a controlled and useful rate over a period of more than one month.

Even further, it is an object of the present invention to provide a system in which both the mechanical and the drug release properties can be tailored and optimized independently.

It is a further object of the invention to provide a method to control reproductive function and further to suppress oestrus in a female non-human mammal which comprises the steps of positioning a drug delivery system of the subject invention within the vaginal tract and retaining the system within the vaginal tract for at least about 7 days.

It is another object of the invention to provide a method to optimize reproductive performance in a female non-human mammal which comprises the steps of positioning a drug delivery system of the subject invention within the vaginal tract and retaining the system within the vaginal tract for at least about 7 days.

In accordance with the present invention a helically-shaped medicated veterinary system suitable for delivery of a drug to the vaginal cavity of a female non-human mammal is provided, comprising a three-layered polymer fibre comprising a polymer core, a polymer intermediate layer comprising a drug covering the core, and a polymer skin covering the intermediate layer,

- the system comprises a number of loops in the range of more than 1 to 10,
- the outer dimension of the system - when inserted into said cavity - substantially coincides with the inner dimension at the cervix point of said cavity, and
- the polymeric material in said polymer core, said polymer intermediate layer and said polymer skin comprises ethylene-vinyl acetate copolymer.

A helically-shaped drug delivery system according to the invention may be applied in vaginal cavities in female non-human mammals.

The present invention will now be described in more detail for an embodiment wherein the system has the form of a helically-shaped drug delivery system for vaginal application. In the context of the present invention "vaginal spring", "spring " , "helically-shaped medicated veterinary system" and "a helically-shaped drug delivery system" are used interchangeably.

Since the invention pertains to a drug delivery device for intra-vaginal use in a female non-human mammal, in particular in a companion or a farm animal such as a horse (mare), a swine (sow or gilt) or a head of cattle (cow or heifer), its use is focused typically on female indications including contraception, control of reproductive function, maintenance of pregnancy, suppression of oestrus, optimization of reproductive performance and regulation of ovarian function allowing to use artificial insemination, IVF (in-vitro fertilisation) related technologies and embryo transfer. Zoo-technical indications like optimization of growth patterns and of meat quality may also be obtained by using the vaginal route of delivery.

Control of reproductive function includes synchronization of oestrus and ovulation of groups of female non-human mammals during the breeding season (for species which have a breeding season) as well as induction and synchronization of oestrus and ovulation in groups of female non-human mammals which are not cycling at the time of treatment (non breeding season, post partum anoestrus). Control of reproductive function further includes suppression of oestrus of non-human performance mammals in which oestrus negatively interferes with performance, such as performance mares in which oestrus will negatively interfere with racing, jumping or showing.

Optimize reproductive performance includes improved fertility results associated with the precise timing of ovulation (this allows to do artificial insemination a few hours before ovulation). It further includes prevention of early embryonic mortality in female non-human mammals which have sub-optimal progesterone concentrations following ovulation.

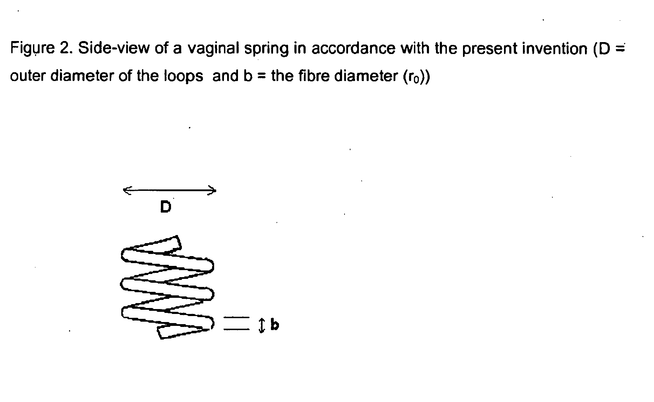

In the context of the present invention, with helically-shaped is meant the shape of a fibre helix with more than one loop and the two ends which are not joined together (Figure 2). The loops of the system embrace many shapes, such as oval, ellipse, toroidal, triangular, square, hexagon, octagon, and the like and combinations thereof. The substantial circular shape of the loops is preferred.

The loops of the coiled spring may be entwined.

Vaginal rings are not helically-shaped and were shown not to be retained efficiently in the vaginal cavity of non-human mammals.

Medicated means loaded with a drug. In production of a medicated three-layered polymer fibre a drug can be loaded in the intermediate layer only, in the intermediate layer and the core, in the intermediate layer and the skin or in the intermediate layer, core and skin. The medicated fibre is loaded with at least one drug.

The expression drug as used herein broadly includes one or more compounds that can be delivered in effective amounts to produce a therapeutic effect. In a preferred embodiment the drug is a steroid. The steroids include progestogenic, androgenic and estrogenic substances. In a more preferred embodiment the drug is selected from the group consisting of progesterone, trenbolone acetate, estradiol, altrenogest and melengestrol acetate (MGA). In a most preferred embodiment the drug is altrenogest.

In one embodiment drugs with a saturation solubility of larger than 0.03% by weight in a polyethylene vinyl acetate matrix, containing 28% vinyl acetate by weight (EVA 28), are preferred. In another embodiment drugs with a saturation solubility of > 0.3% by weight are preferred, in yet another embodiment drugs with a saturation solubility of >1.0% by weight are preferred and in even another embodiment drugs with a saturation solubility of >3.0% by weight are preferred. Solubility can be measured as described in Laarhoven, J.A.H., et al. (2002). International Journal of Pharmaceutics 232, page 165. Briefly, films of poly-EVA were immersed in saturated aqueous solutions of drug at 25 and 37°C. After equilibrium was reached, the films were analyzed on drug content by means of HPLC.

In one embodiment drugs with a molecular weight of < 1000 Dalton are preferred, in another embodiment drugs with a molecular weight of < 700 Dalton are preferred, in yet another embodiment drugs with a molecular weight < 500 Dalton are preferred and in even another embodiment drugs with a molecular weight of < 400 Dalton are preferred.

In another embodiment, the amount of drug contained in the intermediate layer is from 1-70 wt%, in a further embodiment it is from 10-70 wt%, in a still further embodiment it is from 25-65 wt%, and in yet another embodiment it is about 35 to 45 wt %.

When a drug in the manufacturing process of the spring is loaded in the intermediate layer or in the core, the drug diffuses during the production process and/or during storage of the spring to the other polymer layer(s) up to equilibrium concentration.

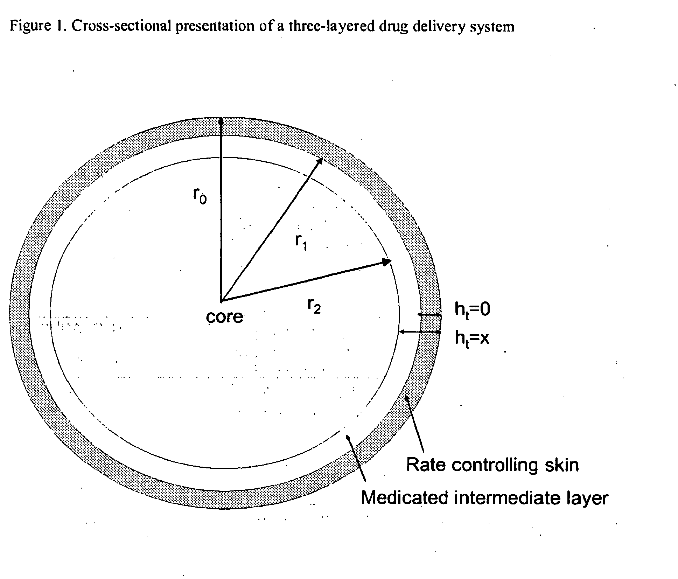

Cross-sectional presentation of a fibre of a three-layered drug delivery system is presented in Figure 1. The shape of the cross-section is substantially circular or substantially elliptical. The substantial circular shape of the cross-section is preferred.

The helically-shaped vaginal spring may have a large number of loops to provide a surface area for delivering an effective amount of drug at a controlled rate over a prolonged period of time. It is an advantage of the helically-shaped spring that just prior to insertion, part of the loops of the spring can be cut off to a predetermined length in order to fine-tune release rate of the system to the, weight of the female mammal.

To improve accommodation by adapting position of the system in the vaginal cavity, the system comprises a number of loops in the range of more than 1 to 10, preferably in the range of 1.5 to 5, more preferably in the range of 2 to 5.

The polymeric material in the polymer core, the polymer intermediate layer and the polymer skin comprises the thermoplastic ethylene-vinyl acetate copolymer (EVA). EVA is used in the three-layered spring according to the invention due to its excellent mechanical and physical properties, including its flexibility. The polymeric material may be a mixture of ethylene-vinyl acetate copolymer and any extrudable thermoplastic polymer or elastomer material suitable for pharmaceutical use, such as low density polyethylene, polyurethanes, and styrene-butadiene copolymers. The polymeric material of the core, the intermediate layer and the skin comprises preferably at least 50 % w/w, more preferably at least 80 % w/w and most preferably at least 95% w/w of ethylene-vinyl acetate copolymer. EVA copolymer used for the core, the intermediate layer and the skin may be of the same or different grade. The copolymer can be any commercially available ethylene-vinyl acetate copolymer, such as the products available under the trade names: Elvax, Evatane, Lupolen, Movriton, Ultrathene, Ateva, and Vestypar. These ethylene-vinyl acetate copolymers are available in different grades with respect to the amount of vinyl acetate present in the copolymer.

For example, EVA 28 (Ateva 2820A) is a copolymer having a vinyl acetate content (VA) of approximately 28%; EVA 33 (Ateva 3325AC) contains approximately 33% VA; EVA 18 (Ateva 1821 A) contains approximately 18% VA and EVA 9 (Ateva 1070) contains approximately 9% VA.

In another embodiment the core of the three-layered spring comprises ethylene-vinyl acetate copolymer with a vinyl acetate content of less than 18% and preferably less than 10%.

In a further embodiment, both the core and intermediate layer are made of the same grade of ethylene-vinyl acetate copolymer. The thickness of the skin and the vinyl acetate content of the skin influence the release rate of the drug. The thinner the skin and the higher the vinyl acetate content of the skin, the higher the release rate of the drug.

In one embodiment, EVA copolymers having a vinyl acetate content of from 0 to 40% are used. In another embodiment, EVA copolymers having a vinyl acetate content of from 6 to 40% are used. In yet another embodiment, EVA copolymers having a vinyl acetate content of from 6 to 33% are used. In still another embodiment, EVA copolymers having a vinyl acetate content of from 9 to 33% are used. In a further embodiment, the core is made of EVA 9 or 28. In a still further embodiment, the skin is made of EVA copolymers having a vinyl acetate content of from 6 to 33% or from 9 to 33%, for example, EVA 9, EVA 15, EVA 18, EVA 28 or EVA 33. In a further embodiment, the skin is made of EVA 33. It is known in the art that the lower the vinyl acetate content of the EVA copolymers used, the higher the stiffness of the vaginal spring made therefrom. Moreover, a larger fibre diameter will also result in less flexibility.

The outer dimension of the system substantially coincides with the inner dimension of the vagina at the cervix point of the vagina. The term "substantially coincides" in this context means that after insertion of the system at the cervix point, its helical shape and mechanical properties including flexibility provide the desired coincidency and accomodation of the outer dimension of the system with the vaginal wall at that point. This comes about by the spring adapting both its outer configuration and position such that its presence creates a low pressure against parts of the wall of the cavity. The mechanical properties and the helical shape allow the spring to adapt its configuration alongside the direction of its axis, perpendicular towards this axis and all directions in between under the physiological conditions of the vaginal cavity. The properties allow lateral distortion of the helically-shaped spring.

It will be clear that the outer dimension of the system in the form "as delivered" will differ from the outer dimension of the system when inserted into the vaginal cavity. The former may for instance be circular, while the latter will more or to a certain extent adapt to the - compared to a circle - irregular inner shape of the vaginal cavity close to the cervix. This substantive coincidence of the system according to the invention is relevant in moderating the pressure against the interior walls of the vaginal cavity and, as a consequence, in regulating the retention time of the system in the female mammal and in regulating tolerability in terms of irritation and inflammation of the tissue in the vaginal cavity, after insertion of the system. High pressure may provide a high retention rate of the system in the treatment period but also low tolerability. The system according to the invention demonstrates both a high retention rate in the treatment period and a high tolerability as a result of its helical shape and its mechanical properties that are fine-tuned to provide a low pressure against the vaginal walls. The system is designed as such, that the system progressively moves backwards in the cavity.

Fick's law of diffusion governs the release of drugs from a three-layered vaginal spring comprising a polymer skin. Drug release kinetics from a three-layered vaginal spring can be of the non-linear or of the essentially zero-order type.

A well appreciated model for describing drug release from a cylindrically shaped reservoir device covered by a rate controlling membrane is (see Figure 1):

$$\frac{dM}{dt} = \frac{2\pi L\, D_p\, K_{p/s}\Delta C}{Ln\, (r_0\, /\, r_1\, )}$$

$L$ = the length of the fibre
$D_p$ = the diffusion co-efficient of the compound in a skin polymer
$K_{p/s}$ = partition coefficient of the compound between the skin and intermediate layer polymer
$\Delta C$ = the difference in concentration between the core intermediate layer and the sink
$r_0$ = the fibre diameter (b in Figure 2))
$r_1$ = is the radius of the core plus intermediate layer.

The equation shows that substantial zero-order release rate is obtained when the term on the right-hand side of the equation is constant, i.e. not a function of time. According to this law, the amount of mass transferred over the boundary is an inverse function of the distance across the boundary. It was found that for a constant release rate it is preferred to concentrate the compound in an intermediate layer between a skin and a core. Since the compound is then concentrated in a relatively thin layer, lengthening of the diffusion distance during release is minimal, resulting in a more constant release rate over time (the term $(r_0\, /\, r_1)$ may be considered almost constant). Concentration of the compound in a relatively thin layer or small intermediate layer volume is advantageous for obtaining springs with a low initial drug load. It was further found that in case the compound in the intermediate layer is only present in the dissolved state, the concentration gradient ($\Delta C$) will steadily decrease in time and consequently the release rate dM/dt will decrease (deviate from zero order release kinetics). Therefore, it is preferred to have the compound present in its solid form in a three-layered spring design.

Compared to a two-layered system, the three-layered system is advantageous in that both the mechanical and the drug release properties can be tailored and optimized independently. EVA copolymers with a relatively low vinyl acetate content are elected for application in the core to achieve high retention rates in treatment periods and high tolerability. Relatively high vinyl acetate contents can be applied to attain the desired rates of controlled drug delivery to a variety of female non-human mammals and various therapeutic and zoo-technical indications. In the three-layered system according to the invention, the copolymers with a relatively low vinyl acetate content can be used as core material, whereas the drug loaded intermediate layer can comprise copolymers with a relatively high vinyl acetate content. In the system, the material used in the core can be varied in order to tune the mechanical properties without significantly affecting the release rate of drug from the system and reverse, the material in the drug-loaded intermediate layer can be varied to desired drug release rates without significantly affecting the mechanical properties of the system.

In addition to that, the system comprises a polymer skin that avoids direct contact between the drug-loaded intermediate layer and the vaginal mucosa, thus having the advantage of reducing the risk of burst release from the drug-loaded intermediate and local irritation due to direct contact with the drug.

Further, three-layered springs have an efficient design for obtaining springs with a low initial drug load. Moreover, in three-layered springs the thickness of the skin and intermediate can be varied as well as the skin material of the springs. In this way the time period in which a therapeutically effective release rate is sustained can be modified as such, that low residual contents of drug in the spring can be obtained at the end of that period by exhaustion of the intermediate layer. Additionally, in three-layered springs where a drug in the intermediate layer only is sufficient to obtain the required drug delivery kinetics, efficient use of the drug can be advantageously further increased by using in the core polymer grades with very low solubility properties for that drug. The high efficiency in delivered drug is advantageous not only from an economical but also from an ecological point of view.

A helically-shaped spring according to the invention has an efficiency in delivered drug of at least 55% and preferably of at least 70%.

In a helically-shaped spring according to the invention, the skin is an ethylene-vinyl acetate copolymer comprising skin having a thickness ranging from 40 to 300 $\mu$m and a vinyl acetate content ranging from 5 to 35%, and more in particularly the skin comprises ethylene-vinyl acetate copolymer with a 25 to 35% vinyl acetate content. Such a skin has excellent drug solubility and diffusion properties, allowing desired rates of controlled drug delivery to a variety of female non-human mammals and various therapeutic and zoo-technical indications during a prolonged period of time. In a helically-shaped spring according to the invention, the core body is advantageously comprising an ethylene-vinyl acetate copolymer with a 2 to 30%, preferably 5 to 15% and more preferably 8 to 11% vinyl acetate content. The percentage of vinyl acetate can be established using potentiometric titration, IR and NMR as described in various textbooks on this subject matter.

The vaginal spring of the present invention can be manufactured by the known process of extrusion, such as co-extrusion and blend extrusion. To obtain the material for the medicated fibre, core or intermediate layer, drug is mixed with an EVA copolymer. The major step in the mixing process is blend extrusion. Subsequently, the drug/EVA copolymer mixture (i.e. intermediate layer comprising a drug) is co-extruded with the core and skin materials into a three-layered fibre. The three-layered fibre thus-obtained is cut into pieces of a desired length and each piece is assembled to a spring-shaped device in any suitable manner known to the person skilled in this art. The springs are then packed, for example in a suitable sachet, optionally after being sterilized or disinfected.

A person skilled in the art of extrusion will have no difficulty in finding the optimal processing conditions, such as determining the extrusion temperature, extrusion speed, and air gap, for making a three-layered fibre containing drug on the basis of methods and procedures known in the art and the description and examples given in this application. A suitable temperature for blend extrusion of the drug/EVA copolymer mixture lies in the range of from 80°C to 170°C, e.g. approx. 105°C. Suitable temperatures for co-extrusion of the three-layered fibre lie in the range of from 80°C to 170°C, e.g. from 105°C to 130°C.

The extrusion temperature is preferably below the melting temperature of the drug.

This is to avoid melting of the drug during extrusion with, as a consequence, phenomena like delayed crystallization. For altrenogest the extrusion temperature is therefore preferably below approximately 118 °C.

The vaginal spring according to the present invention can be manufactured in any practical size. The system can be shaped for delivery of a drug to a vaginal cavity of a female non-human mammal, in particular a companion or a farm animal such as a horse (mare), a swine (sow or gilt) and a head of cattle (cow or heifer).

In one embodiment of the invention "as delivered" for mares, the spring has a fibre diameter in the range of about 4.0 and 8.0 mm, preferably in the range of 4.5 to 6.5 mm. In a further embodiment, the outer diameter of the loops is in the range of about 50 and 90 mm, preferably in the range of about 65 and 90 mm. In one embodiment "as delivered" for swine, the spring has a fibre diameter in the range of about 4.0 and 7.0 mm, preferably in the range of about 4.5 and 6.5 mm. In a further embodiment for gilts, the outer diameter of the loops is in the range of about 25 and 60 mm. In another embodiment for sows, the outer diameter of the loops is in the range of about 35 and 70 mm.

A preferred embodiment of the invention is for placing in the vagina of mares, sows gilts, cows or heifers.

The subject invention provides a method of manufacturing the three-layered drug delivery system of the subject invention with drug loaded in the intermediate layer, comprising:

(i) producing a medicated homogenous polymer intermediate layer granulate;
(ii) co-extruding a polymer core granulate and the intermediate layer granulate with a polymer skin granulate to form the three-layered drug delivery system.
(iii) collecting the fibre on a reel to form a helically shape and subsequently cutting the fibre to a helically-shaped spring or coiling a spring off-line from a fibre.

The production of the medicated homogeneous polymer intermediate layer granulate comprises:

    a. grounding the polymer;
    b. dry powder mixing the grounded polymer with the drug to be loaded in the intermediate layer;
    c. blend extruding the resulting powder mixture;
    d. cutting the resulting medicated polymer strands into granules, thereby obtaining an intermediate layer granulate;
    e. lubricating the intermediate granulate with a lubricant.

EXAMPLE 1: Preparation of the three-layered vaginal spring and analyses of the in-vitro drug release rates

Ten three-layered fibres were prepared (A1-A2, B1-B7 and C1). Each of the fibres was made according to the following procedure.

*Drug-loaded polymer granulate*

Two subsequent mixing steps were performed to mix the altrenogest homogeneously through the polymer (ethylene vinyl acetate containing 33% vinyl acetate, EVA33). In the first step, dry powder mixing was performed with drug and polymer powder. The drug was mixed with the polymer powder in a stainless steel drum using a Rhönrad (Barrel-hoop principle) with a fixed rotation speed of approximately 47 rpm for 60 minutes. This first powder mixing step was performed to mix polymer and drug for the intermediate layer (polymer powder and altrenogest). Subsequently the homogenized powder mixture was blend extruded using a 25 mm co-rotating double screw blend extruder (Berstoff ZE25) and the resulting medicated polymer strands were cut into granules using a Scheer granulator. According to this process a batch intermediate layer granulate was manufactured. After granulation this batch was lubricated with magnesium stereate in order to facilitate the next processing step (co-extrusion). The composition of the granulate batch that was used to manufacture the three-layered fibre, using a co-extrusion process, is described in Table 1 below.

<div align="center">Table 1:</div>

| Material | Composition (%) |
| --- | --- |
| Altrenogest | 40 |
| EVA 33 ° | 59.9 |
| Magnesium Stereate | 0.1 |
| Total | 100 |
| ° EVA copolymer under tradename Ateva applied. | |

*Three-layer co-extrusion*

A Fourné Tricomponent Monofil Spinning plant tri-co-extruder (25/18/18 mm) was used for trico-extrusion. The three extruders were connected with a three-compartment spinning block (Fourné) with three separate spinning pumps (to control the volume flow rate (melt flow) of each layer). The three melt flows were combined in a spinneret resulting in a fibre with three layers. A capillary of 4.2 mm was used. The fibres were extruded at extrusion temperatures of 95 °C (intermediate layer), 100 °C (skin layer) and 130 °C (core). The spin block was set at 105 °C.
The spinning rate was tuned to obtain the desired fibre diameter of 5.0 mm, and the desired layer thickness for skin and intermediate layer was obtained by adjustment of the spinning pumps. Each of the three-layer fibre batches (A1-A2 and B1-B7) was produced by using the appropriate spinning rate and spinning pump settings. After approximately 5 minutes of trico-extrusion of each batch, the three-layered fibre was collected on a tubular glass mandrel moving in a translational and rotational way. The helically-shaped spring was thus obtained. The diameter of the fibre was measured at the beginning, in the middle and at the end of the manufacture of each batch using a laser micrometer and was recorded.
The medicated fibres were processed at an extrusion speed of 0.6 m/min and were collected at rotational speeds of 0.55 m/min (batches A and C1) or 1.1 m/min (batches B) (see Table 2).

*Fibre dimensions.*

The fibre dimensions (fibre diameter, intermediate layer thickness and skin thickness) were determined directly after processing on helically-shaped springs with 3 loops. The fibre diameter was determined by means of laser thickness gauge (Zumbach). The intermediate layer and skin thickness were determined using a microscope (Jena). The results for the medicated batches are shown in Table 2 together with the content of altrenogest in the different fibres.

Table 2: Fibre dimensions and altrenogest content of 5.0 mm medicated fibres processed by means of trico-extrusion, at an extrusion speed of 0.6 m/min.

| Batch | Fibre diameter (mm) | Intermediate layer ($\mu$m) | Skin layer ($\mu$m) | Skin polymer | Core polymer° | Altrenogest content (mg/ 157 mm) | Outer diameter (mm) | Number of loops |
|---|---|---|---|---|---|---|---|---|
| A1 | 4.8 | 280 | 153 | EVA 33 | EVA 9 | 263 | 70 | 2 |
| A2 | 5.0 | 132 | 120 | EVA 33 | EVA 9 | 139 | 70 | 2 |
| B1 | 4.9 | 88 | 130 | EVA 33 | EVA 9 | 89 | 40 | 3 |
| B2 | 5.0 | 113 | 131 | EVA 33 | EVA 9 | 125 | 40 | 2 |
| B3 | 5.0 | 164 | 86 | EVA 33 | EVA 9 | 173 | 40 | 3 |
| B4 | 5.0 | 227 | 91 | EVA 33 | EVA 9 | 239 | 40 | 2 |
| B5 | 5.0 | 120 | 189 | EVA 33 | EVA 9 | 110 | 40 | 2 |
| B6 | 5.0 | 137 | 134 | EVA 18 | EVA 9 | 124 | 40 | 2 |
| B7 | 5.0 | 122 | 183 | EVA 28 | EVA 9 | 171 | 40 | 2 |
| C1 | 5.0 | 993 | 97 | EVA 18 | EVA 9 | 784 | 85 | 3 |
| D1 | 6.0 | 100 | 130 | EVA 28 | EVA 9 | 0 | 40 | 2 |
| D2 | 6.0 | 100 | 80 | EVA 33 | EVA 9 | 0 | 40 | 2 |
| D3 | 6.0 | 150 | 130 | EVA 28 | EVA 9 | 0 | 55 | 2 |
| D4 | 6.0 | 150 | 80 | EVA 33 | EVA 9 | 0 | 55 | 2 |
| D5 | 6.0 | 130 | 80 | EVA 33 | EVA 9 | 0 | 64 | 2 |
| ° EVA copolymer grades under tradename Ateva applied. | | | | | | | | |

In-vitro release of altrenogest was performed in 0.9 % sodium laurylsulphate (SLS) with fibres of approximately 15 cm of length. Samples were collected every day for the predetermined time period and analysed. The obtained release rates were then extrapolated in order to evaluate the expected release in the complete springs.
Results for in vitro release of altrenogest from various batches of vaginal springs (Table 2) are shown in Tables 3a, b, c and d. The release rates are calculated from six samples of each kind of helically-shaped spring tested. The presented values are the mean of six springs.
The magnitude of burst release at day 1, the amounts released at day 7, 10, 14 and 30, and the average release of altrenogest over the first 7, 10, 14 or 30 days are shown in Tables 3a, b, c and d.

Table 3a: in vitro release of vaginal springs comprising altrenogest.

| Batch | Day 1 (mg) | Average day(1-30) (mg/d) | Day 30 (mg) | Fibre length (mm) |
|---|---|---|---|---|
| A1 | 65.8 | 21.0 | 13.2 | 470 |

Table 3b: in vitro release of vaginal springs comprising altrenogest

| Batch | Day 1 (mg) | Average day(1-14) (mg/d) | Day 14 (mg) | Fibre length (mm) |
|---|---|---|---|---|
| B3 | 68.5 | 23.3 | 9.8 | 378 |

Table 3c: in vitro release of vaginal springs comprising altrenogest

| Batch | Day 1 (mg) | Average day(1-10) (mg/d) | Day 10 (mg) | Fibre length (mm) |
|---|---|---|---|---|
| A2 | 64.2 | 28.3 | 15.7 | 470 |
| B4 | 45.7 | 17.0 | 10.6 | 252 |
| B5 | 27.7 | 12.8 | 10.4 | 252 |
| B6 | 10.9 | 5.6 | 4.4 | 252 |
| B7 | 22.0 | 11.1 | 7.6 | 252 |

Table 3d: in vitro release of vaginal springs comprising altrenogest.

| Batch | Day 1 (mg) | Average day(1-7) (mg/d) | Day 7 (mg) | Fibre length (mm) |
|---|---|---|---|---|
| B1 | 51.7 | 23.3 | 10.8 | 378 |
| B2 | 34.1 | 16.8 | 10.6 | 252 |

The results (Tables 3a, b, c and d) show that by applying parameters like fibre diameter, intermediate-layer and skin thickness, type of polymer applied and drug content a broad scope of release characteristics were obtained.

EXAMPLE 2: Efficiency three-layered helically-shaped springs in altrenogest delivery

The total amount of drug released by the delivery system at the end of the treatment time period in percentage of the initial drug load is expressed as efficiency. High efficiency is advantageous not only from an economical but also from an ecological point of view, because it implies a lower residue of active drug in the device after use. In the three-layer spring the skin and intermediate layer thickness as well as the skin material permit to tune the resulting release rate and thus the efficiency of the springs for each particular application. In particular, it is possible to produce systems for which the last day of use corresponds to the time at which a sharp decrease in release rate becomes evident and thus to almost complete depletion of the intermediate layer.
Efficiency of altrenogest is calculated from the remaining content of altrenogest after use in vivo from the embodiments A1, A2, B1, B3 and B5 (Composition: Table 2). The efficiency is at least the percentage as indicated in Table 4.

Table 4: Efficiency (%) of helically-shaped springs (Composition:Table 2).

| Batch | Efficiency (at least) of altrenogest spring (%) | Skin layer° ($\mu$m) | Skin polymer° | Altrenogest content (mg/157 mm) |
|---|---|---|---|---|
| A1 (mares) | 73 (30 days)* | 153 | EVA 33 | 263 |
| A2 (mares) | 62 (10 days)* | 120 | EVA 33 | 139 |
| B1 (sows) | 72 (7 days)* | 130 | EVA 33 | 89 |
| B3 (gilts) | 72 (14 days)* | 86 | EVA 33 | 173 |
| B5 | 72** | 189 | EVA 33 | 110 |
| ° EVA copolymer grades under tradename Ateva applied. * duration of treatment **Calculated from in vitro results | | | | |

The efficiency obtained with the present springs is improved compared to the 60% efficiency observed with the PRID® described in US 3,892,238. It is also higher than the release efficacy claimed for recent CIDR® inserts (62% of the original drug load) (M.J. Rathbone, J. Control. Rel. 85 (2003) 105-115). The springs according to the invention show high efficiency in delivering drug.
The clinical efficacy of some of the described embodiments was also determined by evaluating the retention, local tolerance and the clinical efficacy (i.e. estrus synchronization once the spring is removed) of the vaginal springs in vivo. Results are reported in Table 5. The composition of batches A2, B1, B3 and C1 can be found in Table 2.

Table 5. Clinical evaluation of the vaginal springs.

| Batch | Retention in situ (% days/ animal) | Vaginal discharge | Oestrus synchronization (% animals) |
|---|---|---|---|
| A2 (mares) (n = 4) | 100 (10 days*) | none | 100 |
| B1 (sows) (n = 7) | 100 (7 days*) | Not significant (day 0.3 to 1.7) | 100 |
| B3 (gilts) (n = 12) | 85 (14 days*) | Not significant (day 0.3 to 1.7) | 100 |
| C1 (mares) (n = 4) | 100 (120 days*) | None | Not applicable** |
| * duration of treatment<br>** The clinical indication for 120 days is suppression of oestrus | | | |

EXAMPLE 3: Retention rates and tolerance of vaginal springs in mares

Helically-shaped springs according to this invention were inserted deep inside around the cervix of the vagina of mares. The system could be easily inserted in the vaginal cavity. The presence of vaginal discharges and irritation was assessed in 8 mares treated either 10 or 30 days with three-layered altrenogest loaded helically-shaped springs of the batches A1 and A2 (Table 5).

Table 6: Helically-shaped springs manufactured of three-layered fibres comprising altrenogest in intermediate layer (used in mares)

| Batch | Fibre diameter (mm) | Outer diameter of loop (mm) | Number of loops | Intermediate layer ($\mu$m) | Skin layer ($\mu$m) | Skin polymer | Altrenogest content (mg/ 157 mm) |
|---|---|---|---|---|---|---|---|
| A1 | 4.8 | 70 | 2 | 280 | 153 | EVA 33 | 263 |
| A2 | 5.0 | 70 | 2 | 132 | 120 | EVA 33 | 139 |
| ° EVA copolymer grades under tradename Ateva applied. | | | | | | | |

All devices were retained for the target duration. As a consequence retention rate in the treatment period was 100% with both type of devices. Throughout treatment, springs were observed to progressively move backwards. Local tolerance was very good. No adverse event or vaginal discharge was detected.

EXAMPLE 4: Retention rates and tolerance of vaginal springs in gilts

Helically-shaped springs according to this invention were inserted deep inside around the cervix of the vagina of swine. The presence of vaginal discharges and irritation was assessed in 12 gilts treated for 7 or 14 days with three-layered altrenogest loaded helically-shaped springs of the batches B1 to B4. (Table 7).

Table 7: Helically-shaped springs manufactured of three-layered fibres comprising altrenogest in intermediate layer (used in swine)

| Batch | Fibre diameter (mm) | Outer diameter of loop (mm) | Number of loops | Intermediate layer ($\mu$m) | Skin layer ($\mu$m) | Skin polymer° | Altrenogest content (mg/ 157 mm) |
|---|---|---|---|---|---|---|---|
| B1 | 4.9 | 40 | 3 | 88 | 130 | EVA 33 | 89 |
| B2 | 5.0 | 40 | 2 | 113 | 131 | EVA 33 | 125 |
| B3 | 5.0 | 40 | 3 | 164 | 86 | EVA 33 | 173 |

(continued)

| Batch | Fibre diameter (mm) | Outer diameter of loop (mm) | Number of loops | Intermediate layer (μm) | Skin layer (μm) | Skin polymer° | Altrenogest content (mg/ 157 mm) |
|---|---|---|---|---|---|---|---|
| B4 | 5.0 | 40 | 2 | 227 | 91 | EVA 33 | 239 |
| ° EVA copolymer grades under tradename Ateva applied. | | | | | | | |

B3 and B4 springs were used for the 14 days studies, and were retained for 12 +/- 3 and 14 +/- 1 day respectively. Their respective retention rate at 14 days was 67 and 83%. All gilts which retained their device for 14 days displayed a synchronized oestrus 5 days following spring removal.
B1 and B2 springs were retained for 7 days in 100% of the gilts. Irrespective of the duration of treatment, vaginal discharges were uncommon, and when detected always short lived.

EXAMPLE 5: Correlation between the in vivo and in vitro release rate from three-layered spring systems loaded with 40% w/w altrenogest.

Six batches (embodiments A1, A2, B1, B2, B3 and B4) medicated with altrenogest were prepared by means of trico-extrusion. The obtained three-layered springs were used for a pharmacokinetic study in mares and swine. The composition of the batches is illustrated in Table 10 below. All the batches had a core of Ateva 1070 (EVA 9).

Table 10: Helically-shaped springs manufactured of three-layered fibres comprising altrenogest in intermediate layer for an in vivo study in mares and swine.

| Batch | Initial altrenogest content (mg/ unit) | Intermediate layer (μm) | Fibre length (mm) | Skin layer (μm) | Skin ° polymer | Core ° polymer | Outer loop diameter (mm) | Number of loops |
|---|---|---|---|---|---|---|---|---|
| A1 | 788 | 280 | 470 | 153 | EVA 33 | EVA 9 | 70 | 2 |
| A2 | 418 | 132 | 470 | 120 | EVA 33 | EVA 9 | 70 | 2 |
| B1 | 214 | 88 | 378 | 130 | EVA 33 | EVA 9 | 40 | 3 |
| B2 | 201 | 113 | 252 | 131 | EVA 33 | EVA 9 | 40 | 2 |
| B3 | 402 | 164 | 378 | 86 | EVA 33 | EVA 9 | 40 | 3 |
| B4 | 384 | 227 | 252 | 91 | EVA 33 | EVA 9 | 40 | 2 |
| ° EVA copolymer grades under tradename Ateva applied. | | | | | | | | |

The in vitro release of altrenogest (n = 6) from each of the medicated springs was analysed for a time period equal to their duration of use in vivo and the average release rate in vitro (mg/day) was calculated.
Medicated springs were inserted in mares (n = 4) and gilts (n=6) for the specified time periods. After retrieval, the residual content of altrenogest in the springs was determined and the average release rate in vivo (mg/day) was calculated. The correlation (in %) between in vivo and in vitro release is derived from the ratio between the average in vitro and average in vivo release rate.
The results are reflected in Table 11.

Table 11: Comparison of the results obtained with three-layered springs used in the in vitro and in vivo experiments.

| Batch/ Animal | In situ time (days) | Residual content altrenogest after retrieval (mg/ unit) | Average release rate in vivo (mg/d) | Average release rate in vitro (mg/d) | Correlation in vivo/in vitro (%) |
|---|---|---|---|---|---|
| A1 (mares) | 30 | 213 | 19.1 | 21.1 | 90 |

(continued)

| Batch/ Animal | In situ time (days) | Residual content altrenogest after retrieval (mg/ unit) | Average release rate in vivo (mg/d) | Average release rate in vitro (mg/d) | Correlation in vivo/in vitro (%) |
|---|---|---|---|---|---|
| A2 (mares) | 10 | 157 | 26.1 | 28.3 | 90 |
| B1 (gilts) | 7 | 60 | 22.0 | 23.3 | 94 |
| B2 (gilts) | 7 | 88 | 16.1 | 16.8 | 96 |
| B3 (gilts) | 14 | 114 | 20.5 | 21.9 | 90 |
| B4 (gilts) | 14 | 181 | 14.5 | 15.3* | 95 |

*In vitro release was followed experimentally for the first 10 days. The values for the successive 4 days were extrapolated.

In all cases, a correlation of approximately 90% is found between the data calculated from the springs used for animal treatment and the data obtained in vitro.

EXAMPLE 6 : Ease of removal of the springs from gilts

Helically-shaped springs according to this invention (Table 2; batches D1 to D4) were inserted close to the cervix of gilts (n=10 per batch of spring). Fourteen days following insertion, springs were removed. Ease of removal was assessed in all gilts by percentage of springs removed within 60 seconds (Table 12).

Table 12. Percentage of springs removed within 60 seconds.

| Batch | Percentage removed |
|---|---|
| D1 | 20 |
| D2 | 70 |
| D3 | 90 |
| D4 | 80 |

EXAMPLE 7 : Retention rates of vaginal springs in sows

Helically-shaped springs according to this invention were inserted close to the cervix of sows (n=10 per batch of spring). The retention rate of springs from batch D4 and D5 was assessed 7 days after insertion.

Table 13: Helically-shaped springs manufactured of three-layered fibres comprising altrenogest in intermediate layer (used in sows)

| Batch | Fibre diameter (mm) | Outer diameter of loop (mm) | Number of loops | Intermediate layer ($\mu$m) | Skin layer ($\mu$m) | Skin polymer | Altrenogest content (mg/ 157 mm) |
|---|---|---|---|---|---|---|---|
| D4 | 6.0 | 55 | 2 | 150 | 80 | EVA 33 | 0 |
| D5 | 6.0 | 64 | 2 | 130 | 80 | EVA 33 | 0 |
| ° EVA copolymer grades under tradename Ateva applied. | | | | | | | |

Retention rate of both batches D4 and D5 was 100%.

EXAMPLE 8: Long term use of vaginal springs in mares

Helically-shaped springs according to this invention were inserted deep inside around the cervix of the vagina in mares. The system could be easily inserted in the vaginal cavity. The presence of vaginal discharges and irritation was assessed in 8 mares treated for 120 days with three-layered altrenogest loaded helically-shaped springs of the batches E1 and E2 (Table 14).

Table 14: Helically-shaped springs composed of three-layered fibres comprising altrenogest in the intermediate layer (used in mares)

| Batch | Fibre diameter (mm) | Outer diameter of loop (mm) | Number of loops | Intermediate layer ($\mu$m) | Skin layer ($\mu$m) | Skin polymer* | Altrenogest content (mg/ 157 mm) |
|---|---|---|---|---|---|---|---|
| E1 | 6 | 75 | 3 | 993 | 97 | EVA18 | 784 |
| E2 | 6 | 75 | 5 | 491 | 174 | EVA18 | 430 |
| ° EVA copolymer under tradename Ateva applied. | | | | | | | |

All devices were retained for the target duration of 120 days. As a consequence retention rate in the treatment period was 100% with both types of devices. Throughout the treatment, springs were observed to progressively move backwards. Local tolerance was very good. No adverse event or vaginal discharge was detected. The release rate observed was mostly flat for the all treatment time period (Table 15).

Table 15: Release rate from helically-shaped springs composed of three-layered fibres comprising altrenogest in the intermediate layer (used in mares)

| Batch | Burst day 1 (mg/ spring) | Average release (day 3-60) (mg/day/spring) | Average release (day 61-120) (mg/day/spring) | Average release (day 2-120) (mg/day/spring) | Release day 120 (mg/spring) |
|---|---|---|---|---|---|
| E1 | 35.8 | 11.5 | 9.3 | 11.7 | 8.1 |
| E2 | 39.8 | 14.1 | 12.5 | 14.5 | 11.4 |

**Claims**

1. A helically-shaped medicated veterinary system suitable for delivery of a drug to the vaginal cavity of a female non-human mammal, **characterized in that**,

   • the system comprises a three layered polymer fibre comprising a polymer core a drug loaded polymer intermediate layer covering the core, and a polymer skin covering the intermediate layer,
   • the system comprises a number of loops in the range of more than 1 to 10,
   • the outer dimension of the system when inserted into said cavity substantially coincides with the inner dimension at the cervix point of said cavity, and
   • the polymeric material in said polymer core, said polymer intermediate layer and said polymer skin comprises ethylene-vinyl acetate copolymer.

2. A drug delivery system according to claim 1, **characterized in that** the system comprises a number of loops in the range of 1.5 to 5.

3. A drug delivery system according to claim 1 or 2, **characterized in that** the helically-shaped system is obtainable by extrusion or by co-extrusion.

4. A drug delivery system according to anyone of claims 1 to 3, **characterized in that** the drug has a solubility at 37°C in a polyethylene vinyl acetate matrix, containing 28% vinyl acetate by weight larger than 0.03%.

5. A drug delivery system according to anyone of claims 1 to 4, **characterized in that** the drug has a molecular weight of less than 900 Dalton.

6. A drug delivery system according to anyone of claims 1 to 5, **characterized in that** ethylene-vinyl acetate copolymers having a vinyl acetate content of from 6% to 40% are used.

7. A drug delivery system according to anyone of claims 1 to 6, **characterized in that** the system has an efficiency in delivered drug of at least 60%.

8. A drug delivery system according to anyone of claims 1 to 7, **characterized in that** the drug is a steroid.

9. A drug delivery system according to anyone of claims 1 to 8, **characterized in that** the drug is altrenogest.

10. A non-therapeutic method to control reproductive function in a female non-human mammal which comprises the steps of

   (i) positioning the drug delivery system of claim 9 within the vaginal tract and
   (ii) retaining the system within the vaginal tract for at least about 7 days.

11. A method according to claim 10 to suppress oestrus in a female non-human mammal.

12. A non-therapeutic method to optimize reproductive performance in a female non-human mammal which comprises the steps of

   (i) positioning the drug delivery system of claim 9 within the vaginal tract and
   (ii) retaining the system within the vaginal tract for at least about 7 days.

13. A method according to any one of claims 10 to 12, wherein the mammal is a companion or a farm animal.

14. A method according to claim 13, wherein the farm animal is a horse, a swine or a head of cattle.

15. A method of manufacturing the three-layered drug delivery system of claim 1 with drug loaded in the intermediate layer comprising:

   (i) producing a medicated homogenous polymer intermediate layer granulate;
   (ii) co-extruding a polymer core granulate and the intermediate layer granulate with a polymer skin granulate to form the three-layered drug delivery system.
   (iii) collecting the fibre on a reel to form a helically shape and subsequently cutting the fibre to a helically-shaped spring or coiling a spring off-line from a fibre.

16. A method according to claim 15 wherein step (i) comprises:

   - (a) grounding the polymer;
   - (b) dry powder mixing the grounded polymer with the drugs to be loaded in the intermediate layer;
   - (c) blend extruding the resulting powder mixture;
   - (d) cutting the resulting medicated polymer strands into granules, thereby obtaining an intermediate layer granulate;
   - (fe) lubricating the intermediate granulate with a lubricant;

**Patentansprüche**

1. Helikal geformtes, Arzneimittel-enthaltendes tiermedizinisches System, das für die Abgabe eines Arzneimittels an die Vaginalhöhle eines weiblichen, nicht-menschlichen Säugers geeignet ist, **dadurch gekennzeichnet, dass**

   • das System eine dreischichtige Polymerfaser umfasst, umfassend einen Polymerkern, eine Arzneimittel-beladene Polymer-Zwischenschicht, die den Kern bedeckt, und eine Polymerhaut, die die Zwischenschicht bedeckt,
   • das System eine Anzahl von Windungen im Bereich von mehr als 1 bis 10 umfasst,

• die Außenabmessung des Systems, wenn in die Höhle eingeführt, im Wesentlichen mit der Innenabmessung der Höhle am Zervixpunkt zusammenfällt, und
• das Polymermaterial des Polymerkerns, der Polymer-Zwischenschicht und der Polymerhaut Ethylen-Vinylacetat-Copolymer umfasst.

2. Arzneimittelabgabesystem gemäß Anspruch 1, **dadurch gekennzeichnet, dass** das System eine Anzahl von Windungen im Bereich von 1,5 bis 5 umfasst.

3. Arzneimittelabgabesystem gemäß Anspruch 1 oder 2, **dadurch gekennzeichnet, dass** das helikal geformte System durch Extrusion oder durch Coextrusion erhältlich ist.

4. Arzneimittelabgabesystem gemäß einem der Ansprüche 1 bis 3, **dadurch gekennzeichnet, dass** das Arzneimittel eine Löslichkeit bei 37 °C in einer Polyethylenvinylacetat-Matrix, die 28 Gew.-% Vinylacetat enthält, von größer als 0,03 % aufweist.

5. Arzneimittelabgabesystem gemäß einem der Ansprüche 1 bis 4, **dadurch gekennzeichnet, dass** das Arzneimittel ein Molekulargewicht von weniger als 900 Dalton aufweist.

6. Arzneimittelabgabesystem gemäß einem der Ansprüche 1 bis 5, **dadurch gekennzeichnet, dass** Ethylen-Vinylacetat-Copolymere mit einem Vinylacetatgehalt von 6 % bis 40 % verwendet werden.

7. Arzneimittelabgabesystem gemäß einem der Ansprüche 1 bis 6, **dadurch gekennzeichnet, dass** das System einen Wirkungsgrad der Arzneimittelabgabe von wenigstens 60 % aufweist.

8. Arzneimittelabgabesystem gemäß einem der Ansprüche 1 bis 7, **dadurch gekennzeichnet, dass** das Arzneimittel ein Steroid ist.

9. Arzneimittelabgabesystem gemäß einem der Ansprüche 1 bis 8, **dadurch gekennzeichnet, dass** das Arzneimittel Altrenogest ist.

10. Nichttherapeutisches Verfahren zum Steuern der Fortpflanzungsfunktion bei einem weiblichen nicht-menschlichen Säuger, umfassend die Schritte:

   (i) Positionieren des Arzneimittelabgabesystems gemäß Anspruch 9 in dem Vaginaltrakt und
   (ii) wenigstens etwa 7 Tage Belassen des Systems in dem Vaginaltrakt.

11. Verfahren gemäß Anspruch 10 zum Unterdrücken des Östrus bei einem weiblichen nicht-menschlichen Säuger.

12. Nichttherapeutisches Verfahren zum Optimieren der Fortpflanzungsleistung bei einem weiblichen nicht-menschlichen Säuger, umfassend die Schritte:

   (i) Positionieren des Arzneimittelabgabesystems gemäß Anspruch 9 in dem Vaginaltrakt und
   (ii) wenigstens etwa 7 Tage Belassen des Systems in dem Vaginaltrakt.

13. Verfahren gemäß einem der Ansprüche 10 bis 12, wobei der Säuger ein Haus- oder Nutztier ist.

14. Verfahren gemäß Anspruch 13, wobei das Nutztier ein Pferd, ein Schwein oder ein Rind ist.

15. Verfahren zum Herstellen des dreischichtigen Arzneimittelabgabesystems gemäß Anspruch 1, bei dem Arzneimittel in der Zwischenschicht enthalten ist, umfassend:

   (i) Herstellen eines Arzneimittel-enthaltenden, homogenen Polymerzwischenschicht-Granulats;
   (ii) Coextrudieren eines Polymerkern-Granulats und des Zwischenschicht-Granulats mit einem Polymerhaut-Granulat, um das dreischichtige Arzneimittelabgabesystem zu bilden;
   (iii) Sammeln der Faser auf einer Spule, um eine helikale Form zu bilden, und anschließend Schneiden der Faser zu einer helikal geformten Feder oder gesondertes Spulen einer Feder aus einer Faser.

16. Verfahren gemäß Anspruch 15, wobei Schritt (i) umfasst:

- (a) Mahlen des Polymers;
- (b) Trockenpulver-Mischen des gemahlenen Polymers mit den Arzneimitteln, die in die Zwischenschicht aufgenommen werden sollen;
- (c) Mischungsextrudieren des erhaltenen Pulvergemischs;
- (d) Schneiden der erhaltenen Arzneimittel-enthaltenden Polymersträngen zu Granulat, um so ein Zwischenschicht-Granulat zu erhalten;
- (fe) Schmieren des Zwischengranulats mit einem Gleitmittel.

**Revendications**

1. Système vétérinaire médicamenteux de forme hélicoïdale convenable pour l'administration d'un médicament à la cavité vaginale d'un mammifère non humain femelle, **caractérisé en ce que**

   - le système comprend une fibre polymère tri-couche comprenant un coeur de polymère, une couche intermédiaire de polymère chargée en médicament recouvrant le coeur, et une peau de polymère recouvrant la couche intermédiaire,
   - le système comprend un certain nombre de boucles dans la plage allant de plus de 1 à 10,
   - la dimension externe du système lors de son insertion dans ladite cavité coïncide sensiblement avec la dimension interne au niveau du point du col de l'utérus de ladite cavité, et
   - le matériau polymère dans ledit coeur de polymère, ladite couche intermédiaire de polymère et ladite peau de polymère comprend un copolymère d'éthylène-acétate de vinyle.

2. Système d'administration de médicament selon la revendication 1, **caractérisé en ce que** le système comprend un certain nombre de boucles dans la plage allant de 1,5 à 5.

3. Système d'administration de médicament selon la revendication 1 ou 2, **caractérisé en ce que** le système de forme hélicoïdale peut être obtenu par extrusion ou coextrusion.

4. Système d'administration de médicament selon l'une quelconque des revendications 1 à 3, **caractérisé en ce que** le médicament possède une solubilité à 37°C dans une matrice de polyéthylène-acétate de vinyle, contenant 28% d'acétate de vinyle en poids, supérieure à 0,03%.

5. Système d'administration de médicament selon l'une quelconque des revendications 1 à 4, **caractérisé en ce que** le médicament possède un poids moléculaire inférieur à 900 Daltons.

6. Système d'administration de médicament selon l'une quelconque des revendications 1 à 5, **caractérisé en ce qu'**on utilise des copolymères d'éthylène-acétate de vinyle ayant une teneur en acétate de vinyle allant de 6% à 40%.

7. Système d'administration de médicament selon l'une quelconque des revendications 1 à 6, **caractérisé en ce que** le système possède une efficacité de médicament administré d'au moins 60%.

8. Système d'administration de médicament selon l'une quelconque des revendications 1 à 7, **caractérisé en ce que** le médicament est un stéroïde.

9. Système d'administration de médicament selon l'une quelconque des revendications 1 à 8, **caractérisé en ce que** le médicament est l'altrenogest.

10. Méthode non thérapeutique de contrôle de la fonction reproductrice chez un mammifère non humain femelle, comprenant les étapes consistant à :

    (i) placer le système d'administration de médicament selon la revendication 9 au sein du tractus vaginal, et
    (ii) maintenir le système au sein du tractus vaginal pendant au moins environ 7 jours.

11. Méthode selon la revendication 10, destinée à supprimer l'oestrus chez un mammifère non humain femelle.

12. Méthode non thérapeutique destinée à optimiser les performances reproductrices chez un mammifère non humain femelle, comprenant les étapes consistant à :

(i) placer le système d'administration de médicament selon la revendication 9 au sein du tractus vaginal, et

(ii) maintenir le système au sein du tractus vaginal pendant au moins environ 7 jours.

**13.** Méthode selon l'une quelconque des revendications 10 à 12, dans laquelle le mammifère est un animal familier ou un animal d'élevage.

**14.** Méthode selon la revendication 13, dans laquelle l'animal d'élevage est un cheval, un porc ou une tête de bovin.

**15.** Méthode de fabrication du système d'administration de médicament tri-couche selon la revendication 1, ayant un médicament chargé dans la couche intermédiaire, comprenant :

(i) la production d'un granulé de couche intermédiaire de polymère homogène médicamenteuse ;

(ii) la coextrusion d'un granulé de coeur de polymère et du granulé de couche intermédiaire avec un granulé de peau de polymère afin de former le système d'administration de médicament tri-couche ;

(iii) la récupération de la fibre sur une bobine afin de former une forme hélicoïdale puis couper la fibre en un ressort de forme hélicoïdale ou torsader un ressort hors ligne à partir d'une fibre.

**16.** Méthode selon la revendication 15, dans laquelle l'étape (i) comprend :

(a) le broyage du polymère ;

(b) le mélange en poudre sèche du polymère broyé avec les médicaments à charger dans la couche intermédiaire ;

(c) l'extrusion en mélange du mélange de poudre résultant ;

(d) le découpage des fils de polymère médicamenteux résultants en granulés, pour obtenir ainsi un granulé de couche intermédiaire ;

(fe) la lubrification du granulé intermédiaire par un lubrifiant.

Figure 1. Cross-sectional presentation of a three-layered drug delivery system

Figure 2. Side-view of a vaginal spring in accordance with the present invention (D = outer diameter of the loops and b = the fibre diameter ($r_0$))

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4237885 A
- WO 9740776 A
- WO 2004105854 A
- US 4292965 A
- EP 0876815 A
- WO 2004103336 A
- US 20030149334 A
- US 3892238 A
- US 20060051391 A

### Non-patent literature cited in the description

- **PALMER.** *Journal of Reproduction and Fertility,* 1979, vol. 27, 263-270
- **J.B., GRIMMET.** *Theriogenology,* 2002, vol. 58, 585-587
- **DUSART, P. et al.** *Proc 9th congress of the World Equine Veterinary Association,* 2006, 239-241
- **HANDLER, J et al.** *Theriogenology,* 2006, vol. 65, 1145-1158
- **M.J. RATHBONE.** *J. Control. Rel.,* 2003, vol. 85, 105-115